# EUROPÄISCHE PATENTANMELDUNG

(11) **EP 2 093 221 A1**
(43) Veröffentlichungstag der Anmeldung: **26.08.2009**
(21) Anmeldenummer: 09000515.8
(22) Anmeldetag: 15.01.2009
(51) Int. Cl.: C07D 301/26

(54) **Verfahren zur Herstellung von Epichlorhydrin aus Glyzerin**

(30) Priorität: 04.02.2008 DE 102008007622
(71) Anmelder: Biopetrol Industries AG, 6300 Zug (CH)
(72) Erfinder: Kanzler, Walter, 8010 Graz (AT)
(74) Vertreter: Lenzing, Andreas

(57) **Zusammenfassung**

Es wird ein Verfahren zur Herstellung von Epichlorhydrin durch Chlorierung von Glyzerin mit Chlorwasserstoff, durch Carbonsäuren katalysiert, in einem Reaktor und einer Destillationseinheit mit anschließender Verseifung des gebildeten Dichlorpropanols durch Natronlauge vorgeschlagen, bei dem
a) das Glyzerin mit gasförmigem Chlorwasserstoff in einer Blasensäule (K10) chloriert wird, wobei
b) der Chlorwasserstoff, das Glyzerin und eine aus der Destillationseinheit (K14, K18) zurückgeführte Mischung aus Chlorpropandiol, sowie Resten von Glyzerin, Dichlorpropanol und Rückständen im Sumpf der Blasensäule (K10) zugeführt werden und
c) am Kopf der Blasensäule eine Mischung bestehend aus Monochlorpropandiol, Dichlorpropanol, Wasser, Resten von Glyzerin und Rückständen abgezogen wird und der Destillationseinheit (K14,K18) zugeführt wird, worauf
d) das abdestillierte Dichlorpropanol und Wasser mit Natronlauge verseift wird.

## Beschreibung

Bei der Spaltung und Umesterung von Triglyzeriden entsteht Glyzerin, in einer Menge von ca. 10 % der eingesetzten Triglyzeride, das wegen seiner hohen Reaktivität der drei OH-Gruppen für chemische Synthesen bestens geeignet ist.

DE 197308 ( Böhringer) beschreibt ein Verfahren, bei dem Glyzerin mit gasförmigem Chlorwasserstoff unter Überdruck und von Essigsäure oder Propionsäure katalysiert zu Dichlorpropanol reagiert, wobei sich aber ein Gleichgewicht mit ca. 75 % Umsatz einstellt.

DE 1 075 103 (VEB Leuna) beschreibt ein Verfahren, bei dem gegebenenfalls unter Anwesenheit von Schwefeliger Säure in einer einstufigen Destillation ein Azeotrop aus Wasser und Dichlorpropanol abdestilliert und im Anschluss mit Kalkmilch verseift wird und danach in einem horizontalen Destillationsrohr ein Azeotrop aus Epichlorhydrin und Wasser abgezogen wird. Bei diesem Verfahren kann Dichlorpropanol nur mit einer Ausbeute von ca. 10 % des eingesetzten Glyzerins gewonnen werden und in der Verseifung entstehen große Mengen von Kalziumchlorid in verunreinigter Form als Rückstand.

WO 2005/021476 A1 (Spolek) beschreibt die Chlorierung von Glyzerin und Monochlorpropandiol in einer Reaktorkaskade, katalysiert durch Essigsäure, unter Abtrennung eines Azeotrops, bestehend aus Reaktionswasser und einem Teil des produzierten Dichlorpropanols. Die Hauptmenge des Dichlorpropanols wird aber nach einer weiteren Destillation wieder in den Reaktor rückgeführt und nur das Sumpfprodukt bestehend aus den Rückständen ausgeschleust. Das Verfahren hat den Nachteil, daß im Kopfprodukt der Destillation noch größere Mengen an Chlorwasserstoff enthalten sind und entweder das abdestillierte Wasser mit dem Chlorwasserstoff bei starkem Unterdruck und niedriger Temperatur nur in einer Kälteanlage kondensiert werden kann oder das im Reaktor gebildete Dichlorpropanol kann nur mit einem erheblichen Rücklauf an Wasser in den Kopf der Rektlflkationskolonne als Azeotrop mit nur ca. 23 % Gehalt an Dichlorpropanol gewonnen werden, was einen enormen Energieverbrauch der Rektifikation verursacht.

Die als Katalysator eingesetzte Essigsäure wird wegen ihres hohen Dampfdrucks in der Rektifikation aus dem Reaktionsgemisch ausgetragen, erhöht bei der Verseifung den Alkali Verbrauch und verunreinigt die Salzlösung.

WO 2005/054167 A1 (Solvay) beschreibt Verfahren zur Chlorierung von verschiedenen Qualitäten von Glyzerin mit Verunreinigungen aus den Herstellungsprozessen von Glyzerin. Als Katalysator wird Essigsäure oder andere Carbonsäuren, insbesondere Adipinsäure und deren Derivate vorgeschlagen. Der Einsatz von verunreinigten Glyzerinen führt aber meist zur Verschlechterung der Ausbeuten und Bildung von großen Mengen an Destillationsrückständen, die in Verbindung mit chlorierten Komponenten nur teuer zu entsorgen sind und daher besser vor der Chlorierung abgetrennt werden.

Aufgabe der vorliegenden Erfindung ist es daher, ein Verfahren zu schaffen, nach dem Epichlorhydrin durch Chlorierung mit Chlorwasserstoff und anschließender Verseifung hergestellt wird, um den Energieeinsatz und die Menge an zu entsorgenden Rückständen auf ein Mindestmaß zu reduzieren.

Diese Aufgabe wird von einem Verfahren mit den Merkmalen des Anspruchs 1 gelöst.

Weil das Glyzerin mit gasförmigem Chlorwasserstoff in einer Blasensäule chloriert wird, wobei der Chlorwasserstoff, das Glyzerin und die aus der Destillationseinheit zurückgeführte Mischung aus Chlorpropandiol, sowie Resten von Glyzerin, Dichlorpropanol und Rückständen im Sumpf der Blasensäule zugeführt werden und am Kopf der Blasensäule die Mischung, bestehend aus Chlorpropandiol, Dichlorpropanol, Wasser, Resten von Glyzerin und Rückständen abgezogen wird und der Destillationseinheit zugeführt wird, worauf das abdestillierte Dichlorpropanol und Wasser mit Natronlauge verseift wird, ist ein relativ geringer Energieeinsatz nötig und es fallen nur geringe Mengen an Rückständen an.

Wenn weiter das Verseifen mit Natronlauge mehrstufig, insbesondere zweistufig erfolgt , indem in der ersten Stufe Natronlauge in vorzugsweise stöchiometrischem Verhältnis zugegeben wird und der Großtell des dadurch gebildeten Epichlorhydrins in einer Rektifikationskolonne abdestilliert wird und in einer zweiten Stufe unter Nachdosierung von Natronlauge das restliche Dichlorpropanol praktisch vollständig zu Epichlorhydrin umgesetzt und in einer Strippkolonne, vorzugsweise Rektifikationskolonne, abdestilliert wird, wird der Materialverbrauch weiter reduziert. Das Verfahren wird weiter optimiert, wenn die verbleibende wässrige Natriumchloridlösung nach einer gegebenenfalls notwendigen Nachreinigungsstufe wieder als Rohstoff für die Chlor-Alkali-Elektrolyse eingesetzt wird.

Es wurde überraschenderweise gefunden, dass der Einsatz von 0,5 - 10 Gew.% und insbesondere 2 - 5 Gew. % Oxalsäure als Katalysator in der Blasensäule die besten Reaktionsgeschwindigkeiten für den Umsatz von Chlorpropandiol zu Dichlorpropanol ergibt und die Menge an gebildeten Rückständen und Nebenprodukten niedrig ist.

Der Einsatz einer Blasensäule als Reaktor für die Chlorierung ist besonders vorteilhaft, da der statische Druck am Sumpf der Blasensäule den Partialdruck von Chlorwasserstoff erhöht und die höhere Löslichkeit in der Reaktorflüssigkeit die Reaktionsgeschwindigkeit erhöht. Es hat sich auch gezeigt, dass bei einem Druck am Kopf der Kolonne von 1 bar Absolutdruck und einer Flüssigkeitshöhe von über 5 m bei Reaktionstemperaturen zwischen 100 und 130 °C am Kopf der Blasensäule praktisch kein Chlorwasserstoff mehr entweicht.

Gleichzeitig ist es wichtig, am Kopf der Blasensäule so viel Menge abzuziehen und in die Destillationseinheit einzuspeisen, dass das Gleichgewicht der Reaktion nicht nach links verschoben wird. Da die Chlorierung zum Chlorpropandiol ca. 20 mal so schnell verläuft wie die Reaktion zum Dichlorpropanol, ist es wichtig, die Wasserkonzentration in der Blasensäule niedrig zu halten, und den Kreislauf über eine erste Strippereinheit, in welcher ein Azeotrop, bestehend aus ca. 23 % Dichlorpropanol und ca. 77 % Wasser mit möglichst hoher Menge bei Normaldruck zu betreiben. Bei einem Druck von 1 bar abs. kann das Azeotrop bei 99 °C leicht kondensiert werden. Das mit dem Reaktionswasser gebildete Azeotrop zieht aber nur ca. 13 Gew. % des insgesamt im Reaktor gebildeten Dichlorpropanols ab. Erfindungsgemäß werden die ca. 87 Gew. % Dichlorpropanol , die in der Strippereinheit nicht abgetrennt wurden, in einer Rektifikationskolonne im Vakuum bei 100 bis 200 mbar abs. abgetrennt. Erfindungsgemäß ist es auch möglich und vorteilhaft, einen Teil des vom Wasser befreiten Reaktionsgemisches direkt in den Sumpf der Blasensäule zurückführen und nur den verbleibenden Teil in die Rektifikationskolonne zu leiten.

Es ist notwendig, einen kleinen Teil des Sumpfproduktes dieser Rektifikationskolonne über eine weitere Vakuumrektifikation zu leiten, um Polymere und Nebenprodukte im Sumpf auszuschleusen.

Durch diese Reaktionsführung wird Dichlorpropanol mit hoher Reinheit gewonnen, das bei der Verseifung keine Nebenprodukte bildet. Dadurch kann die Verseifung vollständig durchgeführt werden und das Azeotrop, bestehend aus Epichlorhydrin und Wasser kann in den erfindungsgemäßen, zwei nachfolgenden Strippkolonnen vollständig abgetrennt werden, so dass die verbleibende Natriumchlorid Lösung hohe Reinheit und eine Konzentration von >30 Gew. % aufweist, je nach Konzentration der eingesetzten Natronlauge.

### Anwendungsbeispiel:

Glyzerin mit einer Konzentration von 99,5 Gew.% , 0,4 Gew. % Wasser und 0,1 Gew. % Fettsäureresten wird mit einem Massenstrom von 2000 kg/h in die Blasensäule K10 im Sumpf gemeinsam mit 1560 kg/h Chlorwasserstoff und dem Retourlauf aus K14 und K18 aufgegeben. Die Blasensäule hat eine Höhe von ca. 15 m und der Füllstand der Flüssigkeit ist etwa bei 10 m. Der Betriebsdruck in der Blasensäule ist 1 bar abs. und die Temperatur zwischen 120 und 130 °C gehalten. Der aufgegebene Chlorwasserstoff wird praktisch zu 100 % umgesetzt, Glyzerin wird zu 99 % zu Chlorpropandiol umgesetzt und die Konzentration von Dichlorpropanol beträgt im Sumpf ca. 12 Gew.%.

Am Kopf werden ca. 25.000 kg/h Flüssigkeit abgezogen und im Stripper K14 werden 1160 kg/h Wasser mit 346 kg/h Dichlorpropanol aus der Flüssigkeit ausgetrieben. Die verbleibende Flüssigkeit wird zu ca. 50 % direkt in den Sumpf der Blasensäule K10 zurückgeführt, während die restlichen 50 % der Flüssigkeit der Rektifikation K18 zugeführt wird, in deren Kopf ca. 2.000 kg/h >99 Gew.% iges Dichlorpropanol abgezogen wird und deren Sumpfprodukt zu 90 % in die Blasensäule K10 rückgeführt wird und die restlichen 10% des Sumpfproduktes der K18 werden der Vakuumrektifikation K60 zugeführt, an deren Kopf nicht reagiertes Chlorpropandiol und restliches Dichlorpropanol abgezogen werden , während im Sumpf ca. 45 kg/h Nebenprodukte vermischt mit ca. 25 kg Chlorpropandiol abgezogen werden. Es werden daher ca. 98 % der theoretischen Ausbeute an Dichlorpropanol aus der Chlorierung abgezogen und der Verseifung zugeführt.

Die Verseifung wird mit ca. 2.750 kg/h 30 Gew.% iger Natronlauge durchgeführt und in der Rektifikationskolonne K20 werden am Kolonnenkopf ca. 1.900 kg/h Epichlorhydrin in Form eines wässrigen Azeotrops mit ca. 26 Gew.% Wassergehalt abgezogen, welches bei der Kondensation in eine Wasserphase und in eine Epichlorhydrinphase zerfällt, wobei die wässrige Phase wieder als Rücklauf aufgegeben wird.

Im Sumpf der Kolonne K20 wird die > 30 Gew. %ige Natriumchloridlösung mit ca. 50 kg/h Dichlorpropanol und Epichlorhydrin Resten abgezogen und unter Zusatz von weiteren 43 kg/h 30 Gew.% iger Natronlauge wird die Verseifung in B26 komplett vollzogen und die Salzlösung wird der Strippkolonne K30 zugeführt. Am Kopf der Kolonne K30 wird wieder ein Azeotrop aus dem restlichen Epichlorhydrin und Wasser abgezogen und im Sumpf wird ca. 4.350 kg/h ca. 28 Gew.%ige Natriumchloridlösung mit nur < 5 kg/h Gehalt an Chlorkohlenwasserstoffen abgezogen, die noch einer Behandlung mit Ozon oder Peroxid unterzogen werden und daraufhin als Rohstoff für die Chlor-Alkali-Elektrolyse eingesetzt werden. Durch Einsatz höher konzentrierter Natronlauge in der Verseifung kann die Solekonzentration noch erhöht werden.

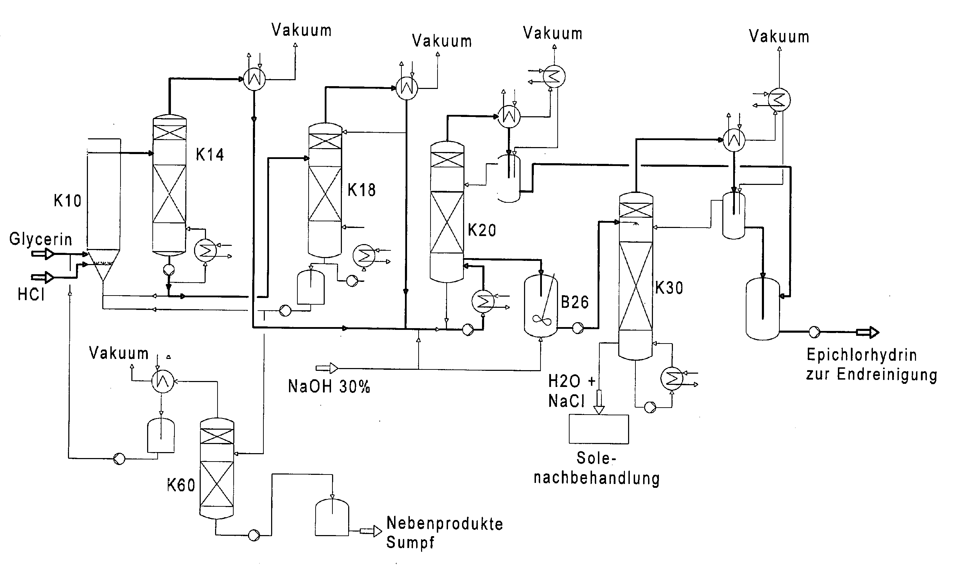

## Patentansprüche

1. Verfahren zur Herstellung von Epichlorhydrin durch Chlorierung von Glyzerin mit Chlorwasserstoff, durch Carbonsäuren katalysiert, in einem Reaktor und einer Destillationseinheit mit anschließender Verseifung des gebildeten Dichlorpropanols durch Natronlauge, **dadurch gekennzeichnet, dass**
a) das Glyzerin mit gasförmigem Chlorwasserstoff in einer Blasensäule (K10) chloriert wird, wobei
b) der Chlorwasserstoff, das Glyzerin und eine aus der Destillationseinheit (K14, K18) zurückgeführte Mischung aus Chlorpropandiol, sowie Resten von Glyzerin, Dichlorpropanol und Rückständen im Sumpf der Blasensäule (K10) zugeführt werden und
c) am Kopf der Blasensäule eine Mischung bestehend aus Monochlorpropandiol, Dichlorpropanol, Wasser, Resten von Glyzerin und Rückständen abgezogen wird und der Destillationseinheit (K14,K18) zugeführt wird, worauf
d) das abdestillierte Dichlorpropanol und Wasser mit Natronlauge verseift wird.

2. Verfahren nach Anspruch 1, **dadurch gekennzeichnet, dass** der Verfahrensschritt d) mehrstufig, insbesondere zweistufig ausgeführt wird, indem
d1) in einer ersten Stufe Natronlauge zugegeben wird und der Großteil des gebildeten Epichlorhydrins in einer Rektifikationskolonne (K20) abdestilliert wird und
d2) in einer zweiten Stufe unter Nachdosierung (B26) von Natronlauge das restliche Dichlorpropanol praktisch vollständig zu Epichlorhydrin umgesetzt und in einer Strippkolonne (K30), vorzugsweise einer Rektifikationskolonne, abdestilliert wird.

3. Verfahren nach Anspruch 1 oder 2, **dadurch gekennzeichnet, dass** nach dem Verfahrensschritt d) bzw. nach den Schritten d1) und d2) in einem Schritt e) verbleibende wässrige Natriumchloridlösung, gegebenenfalls nach einer Nachreinigungsstufe wieder als Rohstoff für die Chlor-Alkali-Elektrolyse eingesetzt wird.

4. Verfahren nach einem der vorhergehenden Ansprüche, **dadurch gekennzeichnet, dass** im Schritt d) bzw. im Schritt d1) die Natronlauge als NaOH in stöchiometrischem Verhältnis zugesetzt wird.

5. Verfahren nach einem der vorhergehenden Ansprüche, **dadurch gekennzeichnet, dass** als Katalysator in der Blasensäule (K10) Oxalsäure zum Einsatz kommt.

6. Verfahren nach einem der vorhergehenden Ansprüche, **dadurch gekennzeichnet, dass** die aktuelle Konzentration an Oxalsäure in der Blasensäule (K10) 0,5 bis 10 Gew.% , vorzugsweise 2 bis 5 Gew. % beträgt.

7. Verfahren nach einem der vorhergehenden Ansprüche, **dadurch gekennzeichnet, dass** die Blasensäule (K10) eine Füllhöhe von mindestens fünf Metern aufweist und die Einsatzstoffe am Boden der Kolonne zugeführt werden.

8. Verfahren nach einem der vorhergehenden Ansprüche, **dadurch gekennzeichnet, dass** die Destillationseinheit zur Abtrennung von Dichlorpropanol und Wasser aus der Blasensäule aus mindestens zwei Destillationskolonnen besteht, wobei die erste Einheit aus einem ein-oder mehrstufigen Stripper (K14) zur Abtrennung eines Azeotrops, bestehend aus Wasser und Dichlorpropanol besteht, wobei das Sumpfprodukt teilweise in die Blasensäule (K10) rückgeführt wird und der verbleibende Teil einer Rektifikationskolonne (K18) zugeführt wird, die im Vakuum arbeitet, wobei am Kopf Dichlorpropanol abgezogen wird und im Sumpf eine Mischung von Chlorpropandiol und Resten von Dichlorpropan, Glyzerin, sowie diversen Rückständen abgezogen wird, die der Blasensäule (K10) wieder zugeführt wird.

9. Verfahren nach Anspruch 8, **dadurch gekennzeichnet, dass** ein Teilstrom des Sumpfproduktes aus der Rektifikation (K18) zur Abtrennung von Dichlorpropanol in eine weitere Vakuumrektifikation (K60) geleitet wird, in deren Kopf Chlorpropandiol von den Hochsiedern wie Polymeren, Diglyzeriden und dergleichen abgetrennt wird, während die Hochsieder als Destillationsrückstand anfallen.
